Europäisches Patentamt

European Patent Office

Office européen des brevets

(19)

(11) Publication number: 0 113 128
A1

(12) EUROPEAN PATENT APPLICATION

(21) Application number: 83113210.5

(22) Date of filing: 29.12.83

(51) Int. Cl.³: C 09 D 5/08
C 09 D 3/24, C 07 C 51/41
C 07 C 53/126

(30) Priority: 31.12.82 PL 239937
31.12.82 PL 239940

(43) Date of publication of application:
11.07.84 Bulletin 84/28

(84) Designated Contracting States:
CH DE FR GB IT LI

(71) Applicant: Instytut Mechaniki Precyzyjnej
ul. Duchnicka 3
Warszawa(PL)

(71) Applicant: Politechnika Gdanska
ul. Majakowskiego 11/12
Gdansk(PL)

(72) Inventor: Szauer, Tadeusz
ul. Worcella 2/4
Gdansk(PL)

(72) Inventor: Iwanow, Jerzy
ul. Neseberska 1/12
Warszawa(PL)

(72) Inventor: Kozlowski, Andrzej
Al. Konfederacji 68
Warszawa(PL)

(72) Inventor: Meczarski, Zbigniew
ul. Szekspira 4/506
Warszawa(PL)

(72) Inventor: Bordzilowski, Jacek
ul. Gospody 66/29
Gdansk(PL)

(72) Inventor: Brandt, Andrzej
ul. Poprzeczna 2
Starogard Gdanski(PL)

(74) Representative: Hansen, Bernd, Dr.rer.nat. et al,
Hoffmann, Eitle & Partner Patentanwälte Arabellastrasse
4
D-8000 München 81(DE)

(54) Corrosion inhibitor for primers and bituminous protectives.

(57) The corrosion inhibitor for primers and bituminous protectives, based on alcalic metal soaps of fatty acids, according to the invention contains vegetable and/or animal fatty acids with the carbon chain length $C_{16}$-$C_{22}$, contains barium hydroxide, contains the product of reaction of vegetable and/or animal fatty acids, with the carbon chain length $C_{16}$-$C_{22}$, with barium hydroxide, led so that first barium hydroxide is introduced into a part of fatty acids and mixed well, and next the mixture of barium hydroxide with acids is introduced to the remaining quantity of acids, and the molar ratios of fatty acids to barium hydroxide amount from 1:0,1 to 1:2.

Corrosion inhibitor for primers

and bituminous protectives

The subject of invention is the corrosion inhibitor for primers and bituminous protectives.

Corrosion inhibitor is used to painting colours, especially the ground only partially removed from the corrosion products. The primers constitute anticorrosive protection of steel constructions of machines and devices working outdoors, ships and so on. Inhibitor serves also in bituminous protectives to preservation of metal articles during storage, especially in condition of direct operation of atmosperic factors, mainly for castings preservation, for agricultural machines chassis conservation, for rolling stock, vehicles and others.

It is known from the polish patent specification nr P.212732 the composition for engine oil enriching containing 1-50 gravimetrics of barium detergent or calcium detergent with the alcalic reserve 60 mg KOH/g along with polymethylosiliconic compound. The composition among others prevents metal parts of engines against various types of corrosion.

The defect of this inhibitor are relatively low inhibitioning properties as concerned atmospheric corrosion. These agents are not sufficient preservation against appearance of black corrosion spots.

The known corrosion inhibitors for primers are such the compounds as fatty amines, the salts of amines and fatty acids, metal soaps and fatty acids soaps, especially the zinc, magnesium and aluminium soaps.

There are known from american patent specification nr 4217142 the corrosion inhibitors for primers being the mixture of magnesium oxide and the magnesium salt of azelate acid $COOH/CH_2/_7COOH$ magnesium azelate in weight ratio from 1:3 to 1:10.

It is known from american patent specification nr 4753924 the corrosion inhibitor for preservation of corroded iron and steel surfaces without the necessity of removing the rust ply. This known inhibitor contains tannin in volume 15-20% of gravimetrics, the cross linking compounds 3-15%, monoethylic ether of ethylene glycol in volume 12-20%, isopropanoll 15-30%, the catalyst and water. The paints containing the known inhibitors do not effectively and totally stop the corrosion process, especially when the fundation is only partially free from the corrosion products.

There are known the inhibitors introduced to bituminous protectives being the fatty acids soaps of such metals as sodium, magnesium or calcium.

The defect of the known inhibitors is not sufficient protection with the coats containing these inhibitors what is due to not sufficient resistance of such coatings to water emulgation which takes place during the direct operation of water on preventive coating.

The objective of the invention is to work out the corrosion inhibitor for primers and for bituminous protectives free from the defects of the known inhibitors, and the composition based on these inhibitors free from the defects of compositions based on the known inhibitors.

The corrosion inhibitor for primers and bituminous protectives based on alcalic soaps of fatty acids in accordance of the matter of invention is characterized of this, that it contains: vegetable and/or animal fatty acids with the carbon chain length $C_{16}$-$C_{22}$, contains the barium hydroxidate, contains the product of reaction leading so, that first the barium hydroxidate is introduced into the part of fatty acids and mixed well, then the mixture of barium hydroxidate with acids is introduced into the remaining part of fatty acids and the molar ratios of fatty acids to the barium hydroxidate amount from 1:0,1 to 1:2.

It is advisable the corrosion inhibitor according to the invention for bituminous protectives to contain along with fatty acids or in place of fatty acids also naphtenosulphonic acids and the barium soaps of naphtenosulphonic acids.

0113128

It is profitable if corrosion inhibitor according to the invention contains the product of reaction of fatty acids with barium hydroxidate, reaction leading so, that first is manufactured the paste made of barium hydroxidate and acids with the weight ratio of barium hydroxidate to the sum of acids amounting about 1:1. The paste is then introduced into the remaining required amount of acids.

It is advisable the corrosion inhibitor according to the invention, used to paints contains the product of reaction of fatty acids with the barium hydroxidate leading so, that barium hydroxide is powdered to granulation from about 10 to 15 micrometers and next it is pasted. The paste is introduced to the remaining required amount of acids.

It is advantegous the corrosion inhibitor according to the invention used for paints to contain the product of fatty acids reaction with $Ba/OH/_2$, leading so, that after introduction the paste to the remaining required amount of fatty acids, reaction runs during about 1h in the temperature from $120^{\circ}C$ to $140^{\circ}C$.

It is desirable the corrosion inhibitor according to the invention used to primers to contain unsaturated fatty acids.

It is advisable the corrosion inhibitor according to the invention used to primers contains unsaturated fatty acids with the iodine number amounting from 5 to 30.

It is profitable the corrosion inhibitor according to the invention used for primers to contain eruic and/or oleinic acid.

It is advisable the corrosion inhibitor according to the invention used for primers to contain the fatty acids with the acid number from 80 to 120.

It is advisable if the corrosion inhibitor according to the invention used for bituminous protectives contains also naphtenosulphonic acids and/or barium soaps of naphtenosulphonic acids.

It is advantageous the corrosion inhibitor according to the invention used to bituminous protectives to contain the barium soaps manufactured in two stages. At the fisrt stage it is made a paste consisting of barium hydroxide and fatty acid and/or naphtenosulphonic acid with the weight ratio of hydroxide to the sum of the acids amounting about 1:1, and at the second stage the paste is introduced into the remaining quantity of acids.

It is desirable if the corrosion inhibitor according to the invention used to bituminous protectives contains also the calcium hydroxide and/or the calcium oxide and the calcium soap of fatty acids of fraction $C_{12}-C_{16}$ and the total amount of fatty acids to the total amount of barium hydroxide and/or barium oxide amounts as per weight from 5:1 to 1:1 respectively.

The inhibitors according to the invention used as additives even in very small quantities along with noted anticorrosive agents used to oils, such the additives as alifatic amines, polyamines, polyalcanoloamines, heterocyclic amines, the salts of amines and of fatty acids, amides, the esthers of ethoxylic amines and of fatty amines improve the inhibitor operation much more than it could be expected from the amount of added inhibitor, considering suming of inhibiting properties. Such a considerable improvement of inhibiting properties may results only from the cooperation of the known compounds with inhibitor according to the invention, which has to be taken as result of a synergism causing a considerable increasement of the inhibiting properties.

After introduction of the inhibitor according to the invention into bituminous protectives it is obtained the bituminous protective with coating resistant to emulgation during the direct operation of water and with a good adhesion of coating against the moisted metal surface. The coatings obtained with help of the inhibitor according to the inventionare resistant to emulgation and durables i.e. with the preventive properties unchanging in time.

Inhibitor according to the invention used to paints is characterized with very high anticorrosive efficiency as concerned the iron surface and also the nonferous metals.

The corrosion inhibitor according to the invention is shown on the example of manufacturing.

Example I. 50 gravimetrics of fatty acids of fraction $C_{16}$-$C_{22}$ with the acid number 100 and the iodine number 20 is introduced into the reactor. The mixer arm is switched on and the temperature is raised to $120^{o}C$. Next, by constant mixing, 10 gravimetrics of barium hydroxide is introduced to the reactor, first powdered in the ball mill to the granulation of order 15-20 micrometers and next rubbed into a paste in a three-roll device with 10 parts of fatty acids of fraction $C_{16}$-$C_{22}$ in the ambiental temperature. The time of dozing the paste 30 minutes. After introduction the whole amount of paste, the temperature is raised to $140^{o}C$ and the process is led during 30 minutes. After this time the contents of the reactor is cooled down as fast as possible, getting the inhibitor useful to primers production, especially in paints based on oil colours, oleo-resinous colours and alkydo colours.

Example II. To the reactor are introduced 50 gravimetrics of saturated cf fatty acids offraction $C_{16}$-$C_{22}$ and 5 gravimetrics of eruic acid. The acid number of acid mixture is 105 and the iodine number is 90. The mixing arm is switched on and the temperature is raised to $130^{o}C$. Next, 12 gravimetrics of barium hydroxide is powdered in the ball mill to the granulation of 10 micrometers. The barium hydroxide is mixed with 12 gravimetrics of fatty acids of fraction $C_{16}$-$C_{22}$ in the ambiental temperature, next it is rubbed into a paste in a three-roll device. Mixing and rubbing is led in the ambiental temperature. The paste is led into reactor being dozed into the hot fatty acid during 30 minutes. After the whole
amount of paste is introduced, the temperature is
raised to 140°C and this temperature and mixing is being
kept during 30 minutes. The contents of the reactor is
cooled down to the ambiental temperature. The manufactured inhibitor is mixed with the primer, obtaining
the paint which gives very high resistance to steel surfaces on which it was put on.

Example III. Into the reactor supplied with the heating
jacket and a high speed mixing arm are introduced 50
gravimetrics of fatty acids of fraction $C_{12}$-$C_{22}$ with
the acid number 120 and 10 gravimetrics of naphtenosulphonic acids. After switching on the mixer arm, the
temperature is raised to 140°C and by slow mixing it is
introduced the paste consisting of barium hydroxide
in a quantity of 15 gravimetrics and of fatty acids
of fraction $C_{12}$-$C_{22}$ in a quantity of 10 gravimetrics
made by rubbing the hydroxide in acids on a three-roll
device. The time of dozing - about 1h. After the dozing
is finished, the mixing and heating is continued in the
temperature of 140°C. Next, the temperature is raised
to 180°C and the reaction runs during subsequent 1 h.
By constant mixing, the contents of the reactor is cooled
down to the ambiental temperature. The inhibitor is
introduced into a bituminous protectives . By means
of introduction the inhibitor according to the invention
into bituminous protectives we obtain the protective
coating with high anticorrosive efficiency which do not
show the sights of emulgation by direct operation
of humidity.

0113128

Steel plates made of steel St3 were protected with the preparation of thickness 200um, on dry, obtained by melting 50 parts of asphalt PS 35/85 in the melting temperature $85^{o}C$, penetration $35^{o}C$ and a temperature of brittleness $+16^{o}C$, with two parts of inhibitor. The ground was protected during 60 cycles of work in the salt chamber. Duration of the 1 cycle was about 8 hours, the temperature inside the chamber $\pm42^{o}C$, concentration of NaCl was about 5% of gravimetrics. The protective kept the tenacity against the ground and did not show any indications of emulgation.

Patent claims

1. Corrosion inhibitor for primers and for bituminous based on alcalic metal soaps of fatty acids, characteristic of this that it contains vegetable and/or animal fatty acids with the carbon chain length $C_{16}$-$C_{22}$, contains barium hydroxide, contains reaction product of vegetable and/or animal fatty acids with the carbon chain length $C_{16}$-$C_{22}$ with barium hydroxide, reaction led so, that first barium hydroxide is introduced into the part of fatty acids and mixed well, and next the mixture of barium hydroxide with acids is introduced to the remaining part of fatty acids, and the molar ratios of fatty acids to barium hydroxide amount from 1:0,1 to 1:2.

2. The corrosion inhibitor according to the claim 1 for bituminous protectives characteristic of this that it contains along with fatty acids or in place of fatty acids, naphtenosulphonic acids and the soaps of naphtenosulphonic acids.

3. The corrosion inhibitor according to the claim 1 characteristic of this that it contains reaction product of fatty acids with barium hydroxide, reaction led so that first is manufactured a paste comprising barium hydroxide and acids, with weight ratio of barium hydroxide to the sum of acids amounting 1:1, which is introduced into the remaining required quantity of acids.

4. The corrosion inhibitor according to the claims 1 and 3 for paints characteristic of this that it contains the product of reaction of fatty acids with barium hydroxide, led so that first barium hydroxide is powdered to granulation from 10 to about 15 micrometers, and next rubbed into a paste which is introduced into the remaining required amount of acids.

5. The corrosion inhibitor according to the claims 1, 3 and 4 for paints, characteristic of this that it contains the product of reaction of fatty acids with Ba/OH/$_2$ led so, that after adding tha paste into the remaining required quantity of acids, reaction runs about 1 h in a temperature from 120$^o$C to 140$^o$C.

6. The corrosion inhibitof for primers according to the claim 1 characteristic of this that it contains unsaturated fatty acids.

7. The corrosion inhibitor for primers according to the claim 1 characteristic of this that it contains eruic acid and/or oleininc acid.

8. The corrosion inhibitor for primers according to the claim 1 characteristic of this that a iodine number of unsaturated fatty acids amounts from 5 to 30.

9. The corrosion inhibitor for primers according to the claim 1 characteristic of this that fatty acids have the acid number from 80 to 120.

European Patent Office

**EUROPEAN SEARCH REPORT**

Application number

EP 83 11 3210

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | US-A-2 858 285 (WILLIAM C. JOHNSON)<br>* Column 5, claim 1 * | 1 | C 09 D 5/08<br>C 09 D 3/24<br>C 07 C 51/41<br>C 07 C 53/126 |
| | --- | | |
| A | US-A-3 313 635 (RAYMOND F. WOLLEK)<br>* Column 11, lines 58-71; column 5, lines 10-45; column 9, example 10 * | 1 | |
| | --- | | |
| A | EP-A-0 037 442 (ICI AMERICAS) | 1 | |
| | ----- | | |

**TECHNICAL FIELDS SEARCHED (Int. Cl. ³)**

C 09 D
C 08 L
C 07 C

The present search report has been drawn up for all claims

| Place of search | Date of completion of the search | Examiner |
|---|---|---|
| THE HAGUE | 04-04-1984 | GIRARD Y.A. |